(19) **Europäisches Patentamt / European Patent Office / Office européen des brevets**

(11) **EP 4 245 340 B1**

(12) # EUROPEAN PATENT SPECIFICATION

(45) Date of publication and mention
of the grant of the patent:
**24.09.2025 Bulletin 2025/39**

(21) Application number: **23160105.5**

(22) Date of filing: **06.03.2023**

(51) International Patent Classification (IPC):
*A61M 16/00* (2006.01)   *A61B 5/389* (2021.01)
*A61B 5/318* (2021.01)   *A61B 5/087* (2006.01)
*A61M 16/04* (2006.01)   *A61M 16/06* (2006.01)
*A61M 16/10* (2006.01)

(52) Cooperative Patent Classification (CPC):
(C-Sets available)
**A61M 16/024; A61B 5/087;** A61B 5/318;
A61B 5/389; A61B 2560/0223; A61M 16/0075;
A61M 16/04; A61M 16/06; A61M 16/0833;
A61M 16/1005; A61M 16/104; A61M 2016/0027;
A61M 2016/0039; A61M 2202/0208;
A61M 2202/0225;   (Cont.)

(54) **SYSTEM FOR SPONTANEOUS BREATH ONSET DETECTION**

SYSTEM ZUR ERKENNUNG DES EINSETZENS VON SPONTANER ATMUNG

SYSTÈME DE DÉTECTION DE DÉBUT D'INHALATION SPONTANÉE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **15.03.2022 US 202217695626**

(43) Date of publication of application:
**20.09.2023 Bulletin 2023/38**

(73) Proprietor: **GE Precision Healthcare LLC
Wauwatosa, WI 53226 (US)**

(72) Inventors:
• **RAMAKRISHNAN, Upasana**
560066 Bangalore (IN)
• **BHAT, Sanketh**
560066 Bangalore (IN)
• **AGARWAL, Etika**
560066 Bangalore (IN)
• **BOHORI, Adnan Kutubuddin**
560066 Bangalore (IN)
• **GAURAV, Ayush**
560066 Bangalore (IN)
• **BOPARAI, Harleen**
560066 Bangalore (IN)

(74) Representative: **Kilburn & Strode LLP
Lacon London
84 Theobalds Road
Holborn
London WC1X 8NL (GB)**

(56) References cited:
EP-B1- 1 056 499     DE-A1- 102015 011 390
JP-A- 2019 501 683     US-B2- 10 758 693

• STROBACH P. ET AL: "Event-synchronous cancellation of the heart interference in biomedical signals", IEEE TRANSACTIONS ON BIOMEDICAL ENGINEERING, vol. 41, no. 4, 1 May 1994 (1994-05-01), USA, pages 343 - 350, XP093066380, ISSN: 0018-9294, DOI: 10.1109/10.284962
• KAHL LORENZ ET AL: "Removal of ECG artifacts from EMG signals with different artifact magnitudes by template subtraction", CURRENT DIRECTIONS IN BIOMEDICAL ENGINEERING, vol. 5, no. 1, 1 September 2019 (2019-09-01), pages 357 - 359, XP093066085, DOI: 10.1515/cdbme-2019-0090

EP 4 245 340 B1

**(Cont. next page)**

(52) Cooperative Patent Classification (CPC): (Cont.)
A61M 2205/702; A61M 2230/04; A61M 2230/60

C-Sets
A61M 2202/0208, A61M 2202/0007;
A61M 2202/0225, A61M 2202/0014

**Description**

BACKGROUND

The subject matter disclosed herein relates to control of a ventilator.

The basic modes of ventilator operation can be divided into sub-categories based on whether a ventilator or a patient initiates a breath. Patient initiated breathes are referred to as spontaneous breaths. When a ventilatory action is triggered by a spontaneous breathing event, it is of the utmost importance that the ventilator starts the inhalation process as soon as the patient tries to breathe. In other words, there should be timing synchrony between the patient effort and the ventilator action. It is estimated that in approximately 50 percent of the breaths there is patient-ventilator asynchrony. Any patient-ventilator asynchrony can lead to patient discomfort, sleep disorders, and delay the weaning of the patient. Currently, there is a delay (e.g., a few hundreds of milliseconds delay) between when the diaphragm of the patient starts muscle activation to when it leads to a change in pressure and flow at the facial region. Thus, there is a need to detect the start of inhalation effort by the patient earlier and to improve the synchronization between the patient and the ventilator during spontaneous breaths.

EP 1 056 499 describes a device and method for triggering ventilatory support in response to myoelectrical activity of a respiration-related muscle, wherein the method comprises a filtering operation to remove electrode motion artifacts, ECG, 50 and 60 Hz interference from the electrical network and high frequency noise from all the EMGdi signals collected by electrodes. US 10,758, 693 describes a method and system for adjusting a level of ventilatory assistance to a patient. A neuro-mechanical efficiency of the patient is determined. The level of ventilatory assist to the patient is determined on the basis of the neuro-mechanical efficiency and a control value and a mechanical ventilation system is adjusted automatically based on the determined level of ventilatory assist to the patient. DE 10 2015 011 390 describes a method and a computer program for ventilating a patient, in particular, but not exclusively, to a concept for a patient's ventilation control based on the patient's combined fatigue indices. JP 2019 501683 describes an apparatus and method for providing data signals indicative of msucle activity relating to inspriatory respiratory effort of a paitent and data signals indicative of mscule acrtivity relating to expiratory respiratory effort of a patient.

BRIEF DESCRIPTION

An aspect of the presently claimed invention is set out in the independent claim. Particular embodiments of this aspect are set out in the dependent claims. Any subject matter contained herein that does not fall within the scope of the appended claims is considered as being useful for understanding the invention.

Certain embodiments commensurate in scope with the originally claimed subject matter are summarized below. These embodiments are not intended to limit the scope of the claimed subject matter, but rather these embodiments are intended only to provide a brief summary of possible embodiments.

In one embodiment not forming part of the presently claimed invention, a computer-implemented method for detecting onset of a spontaneous breath by a patient coupled to a ventilation system is provided. The method includes receiving, at a processor, a pressure signal from a pressure sensor and/or a flow signal from a flow sensor, respectively, coupled to the patient. The method also includes receiving, at the processor, signals from one or more sensors coupled to the patient that measure different physiological parameters from the pressure sensor and the flow sensor. The method further includes detecting, via the processor, the onset of the spontaneous breath by the patient based on the pressure signal and/or the flow signal. The method still further includes synchronizing, via the processor, providing breathing support to the patient via the ventilation system with the onset of the spontaneous breath detected utilizing the pressure signal and/or the flow signal. The method yet further includes while utilizing the pressure signal and/or the flow signal to synchronize providing breathing support, calibrating, via the processor, parameters and thresholds to be utilized in detecting the onset of the spontaneous breath based on the signals from the one or more sensors. The method even further includes after calibration, switching to: detecting, via the processor, the onset of the spontaneous breath by the patient based on the signals from the one or more sensors and synchronizing, via the processor, providing breathing support to the patient via the ventilation system with the onset of the spontaneous breath detected utilizing the signals from the one or more sensors.

In another embodiment useful for understanding the presently claimed invention, a ventilation system is provided. The ventilation system includes a plurality of sensors configured to be coupled to a patient and to generate a signal related to a respiratory function of the patient, wherein the plurality of sensors includes a flow sensor, a pressure sensor, and at least one electromyography (EMG) sensor. The ventilation system also includes a memory encoding processor-executable routines. The ventilation system further includes a processor configured to access the memory and to execute the processor-executable routines, wherein the routines, when executed by the processor, cause the processor to perform actions. The actions include extracting a breathing signature from a respective signal of each sensor of the plurality of sensors. The actions also include estimating or measuring electrocardiogram (ECG) occurrences in an EMG signal received from the at least one EMG sensor. The actions further include detecting onset of a spontaneous breath of the patient based on the breathing signatures extracted from the respective signals of the plurality of sensors and the estimated or measured ECG occurrences.

In a further embodiment useful for understanding the presently claimed invention, a computer-implemented method for real-time calibration of parameters utilized in detecting onset of a spontaneous breath of a patient coupled to ventilation system. The method includes receiving, at a processor, a flow signal, a pressure signal, and an electromyography (EMG) signal from a flow sensor, a pressure sensor, and an EMG sensor, respectively, coupled to the patient. The method also includes detecting, via the processor, the onset of spontaneous breath utilizing the EMG signal using a range of initial values for at least one parameter for an onset detection algorithm. The method further includes detecting, via the processor, the onset of spontaneous breath utilizing at least one of the flow signal and the pressure signal. The method still further includes determining, via the processor, a difference in time in detecting the onset of spontaneous breath in the EMG signal and the at least one of the flow signal and the pressure signal. The method even further includes selecting, via the processor, at least one updated parameter of the onset detection algorithm to be utilized in detecting the onset of spontaneous breath in the EMG signal based at least on the difference in time.

BRIEF DESCRIPTION OF THE DRAWINGS

These and other features, aspects, and advantages of the present subject matter will become better understood when the following detailed description is read with reference to the accompanying drawings in which like characters represent like parts throughout the drawings, wherein:

FIG. 1 is a schematic diagram of a mechanical ventilation system, in accordance with aspects of the present disclosure;

FIG. 2 is a schematic diagram of the processing of sensor signals to determine an onset of a spontaneous breath of a patient coupled to a ventilation system, in accordance with aspects of the present disclosure;

FIG. 3 is a flow chart of a method for patient personalization and real-time adaption for detecting spontaneous breath onset detection, in accordance with aspects of the present disclosure;

FIG. 4 is a flow chart of a method for utilizing breathing signatures and estimating ECG occurrences in detecting onset of a spontaneous breath by a patient, in accordance with aspects of the present disclosure;

FIG. 5 is a schematic diagram of a different processes for extracting a breathing signature from a signal, in accordance with aspects of the present disclosure;

FIG. 6 is a schematic diagram of a process for detecting ECG occurrences and abnormalities (e.g., inverted ECG), in accordance with aspects of the present disclosure;

FIG. 7 is a reference ECG pattern in a signal;

FIG. 8 is a graph illustrating ECG occurrences within an EMG signal, in accordance with aspects of the present disclosure;

FIG. 9 is a graph illustrating ECG abnormalities within an EMG signal, in accordance with aspects of the present disclosure;

FIG. 10 is a schematic diagram of a process for detecting onset of a spontaneous breath in a signal (e.g., EMG signal) utilizing ECG information, in accordance with aspects of the present disclosure;

FIG. 11 is a graph illustrating detecting onset of a spontaneous breath utilizing multiple thresholds and ECG information, in accordance with aspects of the present disclosure;

FIG. 12 are graphs illustrating detecting onset of a spontaneous breath in a flow signal compared to an EMG signal, in accordance with aspects of the present disclosure;

FIG. 13 is a graph illustrating detecting onset of a spontaneous breath in a flow signal compared to an EMG signal, in accordance with aspects of the present disclosure;

FIG. 14 are graphs for EMG and flow data gathered as a calibration dataset, in accordance with aspects of the present disclosure;

FIG. 15 are graphs for time normalization of the moving average of breaths, in accordance with aspects of the present disclosure;

FIG. 16 is a graph of a maximum likelihood function performed for various breaths derived from EMG data for calibration, in accordance with aspects of the present disclosure;

FIG. 17 is a graph illustrating the utilization of modified dynamic thresholds in automated threshold selection on EMG calibration data, in accordance with aspects of the present disclosure;

FIG. 18 is a schematic diagram of an adaptive approach for selecting parameters for EMG-based onset detection algorithms for individual patients, in accordance with aspects of the present disclosure;

FIG. 19 is a graph for quality factor of breath calculated based on initial data collected over a range of values for a parameter (e.g., lower threshold), in accordance with aspects of the present disclosure;

FIG. 20 is a graph for average of time difference in breath onset detection calculated based on initial data collected over a range of values for a parameter (e.g., lower threshold), in accordance with aspects of the present disclosure;

FIG. 21 is a graph for false breath detection calculated based on initial data collected over a range of values for a parameter (e.g., lower threshold), in accordance with aspects of the present disclosure;

FIG. 22 is a graph for false breath detection versus window size over a range of values for a parameter (e.g., lower threshold), in accordance with aspects of the present disclosure; and

FIG. 23 is a flow chart of a method for real-time calibration of parameters utilized in detecting onset of a spontaneous breath of a patient coupled to ventilation system, in accordance with aspects of the present disclosure.

## DETAILED DESCRIPTION

One or more specific embodiments will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present subject matter, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

As may be appreciated, implementations of the present disclosure may be embodied as a system, method, device, or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present disclosure may take the form of a computer program product embodied in one or more computer readable medium(s) having computer-readable program code embodied thereon.

The present disclosure provides systems and methods for detecting the onset of spontaneous breath of a patient coupled to a ventilation system (e.g., mechanical ventilator) utilizing personalized onset detection algorithms for the patient that are adapted in real time. The disclosed embodiments initially (and during recalibration) utilizing signals from pressure/flow sensors to detect onset of a spontaneous breath in a patient and to synchronize providing breathing support to the patient. Subsequently, after calibration (or recalibration), signals from other sensors (e.g., EMG sensors) may be utilized to detect onset of a spontaneous breath in patient (e.g., utilizing parameters determined during calibration/recalibration) and to synchronize providing breathing support to the patient. Sometimes both EMG signals and pressure/flow signals may be utilized to determine the onset of a spontaneous breath. Detecting the onset of the spontaneous breath in one or more of these signals may trigger (e.g., via an onset trigger) providing (and synchronizing) breath support to the patient. The disclosed embodiments may provide for faster and more accurate detection of a patient's effort to breathe. In addition, the disclosed embodiments may improve the synchronization between the ventilator and the patient.

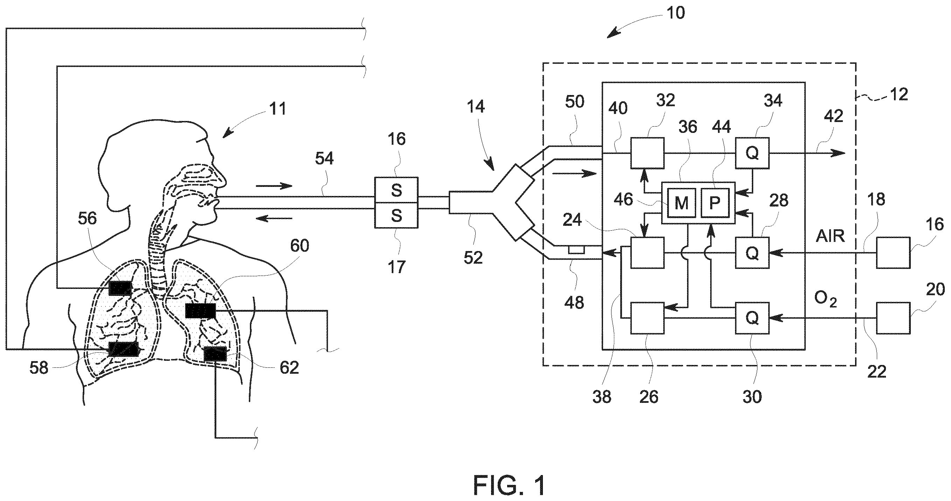

FIG. 1 schematically illustrates an example of a mechanical ventilation system 10. Ventilation system 10 provides a pneumatic circuit that carries breathing gas to and exhaled air from a patient 11 to assist patient 11 in breathing. As described in greater detail below, the ventilation system is configured to detect the onset of spontaneous breath by the patient 11 in a faster and more accurate manner than typical ventilation systems. Ventilation system 10 includes ventilator 12, breathing circuit 14, and sensors 16, 17. The ventilator 12 may operate in a number of modes. The modes include a controlled mode (with the breath is initiated by the ventilator 12), a spontaneous mode (where the breath is initiated by the patient), and a supported mode (where the breath is initiated by both the ventilator and patient). Both the spontaneous mode and the supported mode require synchronization with patient effort.

Ventilator 12 supplies gas, such as air or air including anesthetics, drugs or the like, to patient 11 through breathing circuit 14 and receives exhaled air through breathing circuit 14. In the example illustrated, ventilator 12 receives air from air source 16 through conduit 18 and receives oxygen ($O_2$) from an oxygen source 20 (such as a container of compressed oxygen) through conduit 22. The ventilator 12 includes valves 24, 26, sensors 28, 30, valve 32 and sensor 34 and controller or processing unit 36. Valves 24, 26 control a supply of air and oxygen (the mixture thereof), respectively, through conduit 38 to breathing circuit 14. Sensors 28 and 30 sense or detect the supply of the air and oxygen, respectively, and transmit signals representing such sensed values to processing unit 36.

Valve 32 includes a valve mechanism connected to breathing circuit 14 by conduit 40 so as to control the flow of exhaled air received from breathing circuit 14 to the discharge conduit 42. Sensor 34 includes a device to sense the flow of exhaled air to discharge port 42. Such sensed values for the exhaled air are further transmitted to controller or processing unit 36.

Controller 36 generates control signals controlling the operation of valves 24, 26, and 28. The controller 36 includes one or more processors 44 and a memory 46. The one or more processors 44 executes instructions contained in the memory 46. Execution of the instructions causes the controller 36 to perform steps such as generating control signals. The instructions may be loaded in a random access memory (RAM) for execution by the processing unit from a read only memory (ROM), a mass storage device, or some other persistent storage. In other embodiments, hard wired circuitry may be used in place of or in combination with software instructions to implement the functions described. For example, the processors 44 may be embodied as part of one or more application-specific integrated circuits (ASICs). Unless otherwise specifically noted, the controller 36 is not limited to any specific combination of hardware circuitry and software, nor to any particular source for the instructions executed by the processing unit. In other implementations.

Breathing circuit 14 delivers breathing gas (air, oxygen and possibly other additives such as anesthetics, medicines and the like) from ventilator 12 to patient 11 while also directing exhaled air from patient 11 to system 10 and ventilator 12. Breathing circuit 14 includes inspiratory section or segment 48, expiration segment 50, Y connector 52 and patient segment 54. Inspiratory segment 48 extends from and is pneumatically connected to conduit 38 at one end and Y connector 52 at the other end. Segment 48 delivers gases from conduit 38 to patient segment 54 during forced, assisted or voluntary inhalation by patient 11. Exhalation segment 50 delivers exhaled gases are exhaled air from patient segment 54 to conduit 40. Y connector 52 connects each of segments 48 and 50 to patient segment 54. Patient segment 54 extends from Y connector 52 to patient 11. Patient segment 54 may include devices for pneumatically connecting with patient 11 such as through the nose, mouth or trachea of patient 11.

During inspiration (inhalation), breathing air is delivered through patient segment 54 and into the lungs of patient 11. During expiration or exhalation, expired or exhaled breathing air exits the lungs the patient 11 and is received into patient segment 54. The expired breathing air is communicated or transmitted through patient segment 54, through Y connector 52 and into expiration segment 50. Although not illustrated, in other implementations, ventilation system 10 may include additional devices or systems. For example, in one implementation, system 10 may additionally include a nebulizer positioned between ventilator 12 and inspiratory section 48 to introduce a medical drug or anesthetic agent to breathing air for the patient. In yet other implementations, breathing circuit 14 may include a component such as a humidifier to humidify the breathing air, a heater to heat the breathing air or a water/vapor trap to remove excess moisture from a particular segment or section of ventilation system 10.

In some implementations, ventilator 12 may additionally include a carbon dioxide scavenger which removes carbon dioxide from exhaled air and returns or recycles the air by conducting such recycled air to conduit 18. In one implementation, ventilator 12 utilizes a bellows to pressurize air being supplied to conduit 48. For example, in one implementation, ventilator 12 selectively supplies and withdraws pressurized air to and from an exterior of a bellows assembly. During inhalation, ventilator 12 supplies gas or air to the exterior of the bellows, collapsing the bellows to force gas within bellows to through the carbon dioxide scavenger and to the breathing circuit 14 and the patient's lungs. During exhalation, expelled gas from the patient's lungs passes through valve 32 to fill the bellows. In other implementations, the noted carbon dioxide scavenger as well as the bellows may be omitted.

The ventilation system 10 includes a number of noninvasive sensors to be utilized in determining the onset of a breath (e.g., spontaneous breath) of the patient 11. In certain embodiments, some of the sensors may be invasive (e.g., EMG sensor on a catheter passed through the mouth). Sensor 16 includes a flow sensor to measure flow and direction of gas or air within a passage (e.g., the patient segment 54). Sensor 17 includes a pressure sensor to measure pressure from flow through a passage (e.g., the patient segment 54). In certain embodiments, sensors 16, 17 are part of a device configured to sense or detect pressure and/or flow of air (with or without additives) corresponding to forced, voluntary or assisted inhalation and exhalation by patient 11. In the example illustrated, sensor 16 is located within patient segment 54. In other implementations, sensor 16 may be provided at other locations. For example, in other implementations, sensor 16 may be provided as part of a mouthpiece through which patient 11 inhales and exhales. Sensors 16 and 17 provide signals (e.g., flow and pressure signals) to provide feedback to the controller 36.

The ventilation system 10 includes an EMG sensor 56 (e.g., an EMG patch or surface sensor) disposed on the skin of patient 11 in a location adjacent the upper airway muscles (e.g., on the rear neck region adjacent the posterior cricoarytenoid set of muscles). The EMG sensor 56 measures the action potentials of the respiratory muscle. The EMG sensor 56 provides an EMG signal to the controller 36. The ventilation system 10 also includes an EMG sensor 58 (e.g., an EMG patch or surface sensor) disposed on the skin of patient 11 adjacent the intercostal space. The EMG sensor 58 measures the movement of thoracic/abdominal cavity and/or diaphragm. The EMG sensor 58 provides an EMG signal to the controller 58. In certain embodiments, an EMG sensor may be invasive (e.g., via a catheter passed through the mouth). In certain embodiments, the ventilation system 10 includes one or more ECG sensors 60 disposed on the skin of the patient (e.g., in the chest region and/or intercostal space). The ECG sensor 60 measures cardiac electrical activity. The ECG sensor 60 provides the ECG signal to the controller 58. In certain embodiments, the ventilation system 10 includes one or more additional sensors 62 (e.g., ultrasound, piezoelectric, and/or inductance sensors) disposed on the skin of the patient in the chest or intercostal space. The sensors 62 measure the movement of the thoracic/abdominal cavity and/or the diaphragm. The sensors 62 provides signals to the controller 36.

As discussed in greater detail below, each sensor may be associated with a different onset trigger algorithm for determining the onset of a breath by the patient 11. In certain embodiments, the data collected from the sensors may be fused together to determine the onset of a breath by the patient 11. The sensors may be connected to the controller 36 via a wired or wireless connection. The measurements from the sensors are synchronized.

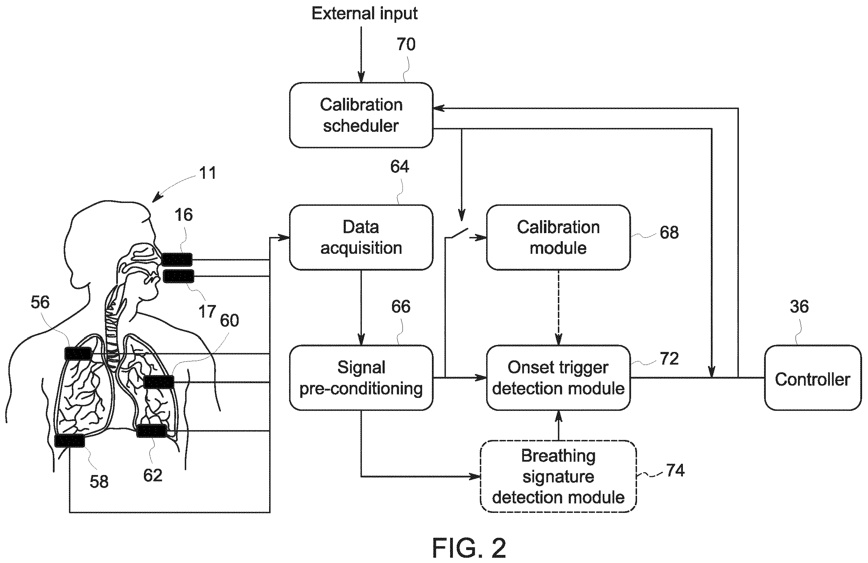

FIG. 2 is a schematic diagram of the processing of the signals to determine an onset of a spontaneous breath of a patient coupled to a ventilation system (e.g., ventilation system 10 in FIG. 1). The processing of the signals to determine the onset of a spontaneous breath of the patient may occur via a number of modules (e.g., stored in and executed by a processing or computing device such as controller 36 in FIG. 1 or a remote processing or computer device coupled to the controller 36). The modules include a data acquisition module 64, a signal pre-conditioning module 66, a calibration module 68, a calibration scheduler module 70, and an onset trigger detection module 72. In certain embodiments, the modules may also include a breathing signature detection module 74 that functions between the signal pre-conditioning module 66 and the onset trigger detection module 72.

The data acquisition module 64 collects or receives the data (via the signals) from the various sensors (the sensors in FIG. 1) coupled to the patient coupled to the ventilation system. The sensors are coupled to the data acquisition module 64 via a wired or wireless connection. The data acquisition module 64 is configured to synchronize the measurements from the sensors.

The signal pre-conditioning module 66 receives the signals from the data acquisition module 64. The signal pre-conditioning module 66 is configured to reduce noise and/or to remove motion artifacts in the signals to improve the signal-to-noise ratio. In certain embodiments, the signal pre-conditioning module 66 is configured to estimate or measure the occurrences of ECG activity in the signals (e.g., signals from a surface EMG sensor and/or ECG sensor). The estimated or measured ECG occurrences may be utilized in the onset trigger detection module to reduce or avoid false triggers of onset detection of a spontaneous breath due to ECG spikes. To estimate or estimate ECG occurrences, machine learning algorithms, peak detection algorithms, and/or QRS complex algorithms may be utilized. In certain embodiments, algorithms utilizing windowed fast Fourier transform algorithms, ECG rate, or wavelets may be utilized to estimate or measure ECG occurrences. In certain embodiments, the estimate or measure of ECG occurrences may occur in the onset trigger detection module 72 (e.g., when the breathing signature detection module 74 is being utilized).

The calibration module 68 is configured to calibrate or determine parameters for the onset detection algorithms utilized on the signals from the various sensors to detect onset of the spontaneous breath by the patient. Examples of parameters include threshold, window size, and variance of baseline entropy of EMG. Calibration accounts for patient to patient variation as well as changing conditions in a patient (e.g., sleeping, change in respiration rate, etc.). Calibration, via the calibration module 68 may include initially (and during re-calibration) utilizing data from the pressure and flow sensors as a truth or baseline for a given period of time while determining or selecting parameters for the onset detection algorithms for the sensors (e.g., EMG sensors) other than the pressure and flow sensors. In certain embodiments, during calibration, initial parameters may be utilized in the onset detection algorithms for the other sensors (e.g., EMG sensors) that may be utilized in the selection of updated parameters for these onset detection algorithms. Optimization, dynamic threshold, and/or maximum likelihood are some of the techniques utilized during calibration. The purpose of the calibration is to minimize the following: average response time (e.g., for providing breathing support in response to detecting the onset of a spontaneous breath), variance in response time, number of false triggers, and the number of missed detections.

The calibration scheduler module 70 is configured to command the calibration module 68 to re-calibrate/compute the parameters for the onset detection algorithms of the onset trigger detection module 72. A calibration schedule may be time-based. For example, calibration may occur after a set time (e.g., 2 hours) passes. A calibration schedule may also be event triggered. For example, a significant change in one or more physiological parameters, receiving a change in a setting (e.g., from medical personnel), or a triggering parameter (e.g., based on a difference between a threshold obtained utilizing a dynamic threshold method and a current threshold).

The onset trigger detection module 72 is configured to detect the onset of a spontaneous breath (e.g., inhalation) in the signals from each sensor. Different algorithms may be utilized for detecting the onset of a spontaneous breath in the signals from the different sensor types. For example, for an EMG signal, moving average, fixed sample entropy, or Hodges-Teager-Kaiser Energy (TKE) operator in combination with thresholding (e.g., single or multiple) may be utilized for onset detection. For signals from the pressure and flow sensors, thresholding (e.g., single or multiple) may be utilized for onset detection. For signals from piezoelectric or inductance sensors, moving average, rate of change, and thresholding may be utilized for onset detection.

In certain embodiments, each sensor based trigger for each of the sensors is associated with its own confidence score based on a variety of factors (e.g., level of noise in the base data, threshold and rate of change in signal, etc.). The onset trigger detection module 72 is configured to determine that the onset of the spontaneous breath is occurring and provides a signal (e.g., a final onset trigger signal to the ventilation system (e.g., to the controller) to provide breathing support to synchronize the breathing support with the onset of the spontaneous breath. In certain embodiments, the onset trigger detection module 72 may provide the onset trigger signal when the onset trigger for one of the sensors reaches and/or surpasses a minimum confidence score.

In certain embodiments, in order to improve accuracy and robustness, the onset trigger detection module is configured to combine or fuse the information from the individual sensors and to utilize heuristics in determining to provide a final onset trigger signal to the ventilation system. For example, a majority-based approached may be utilized where once a majority of the signals from the sensors detect the onset of a spontaneous breath, the onset trigger detection module 72 provides the final onset trigger signal. Heuristics may reduce false triggers. Examples of heuristics may include skipping samples, analyzing slope, ignoring the inhalation phase from the ventilator, or taking into account any health specific abnormality of the patient.

In certain embodiments, in order to improve accuracy and robustness, the onset trigger detection module is configured to multiply the signals from two different sensors before utilizing the onset trigger detection algorithm. For example, after signal conditioning, the flow signal from the flow sensor may be multiplied with the EMG envelope signal prior to looking for the onset trigger.

These approaches reduce false triggers by reducing the robustness to the measurement disturbances. In addition, these approaches enable the utilization of lower thresholds for faster onset detection. While a lower threshold reduces confidence, the fusion of information from the sensors improves confidence overall.

The breathing signature detection module 74 utilizes sensor specific algorithms to extract useful breathing signatures from the signals from each of the sensors. In EMG signals, average signal strength, energy, entropy, or randomness, or peak frequency are examples of a breathing signature. For signals from flow and pressure sensors, scaled signal amplitude, rate of change, or trend estimation are examples of a breathing signature. Examples of algorithms for extracting the breathing signature include moving average (for average signal strength), fixed sample entropy, Hodges-TKEO (for signal energy), and numerical methods for as the difference method to calculate a rate of change of a signal. Different algorithms may be utilized for the different sensors in extracting the breathing signature. For example, for an EMG signal from a first EMG sensor, two different algorithms may be separately applied to the EMG signal (e.g., moving average and sample entropy), while average entropy is applied to an EMG signal from a second EMG sensor and trend estimation is applied to a flow signal from a flow sensor. Extracting a breathing signature involves the detection of the EMG envelope. The parameters (e.g., moving average window size) for the breathing signature extraction algorithms may be pre-fixed or obtained in real-time from the calibration module 68.

As mentioned above, in embodiments where the breathing signature detection module 74 is utilized, the onset detection module 72 is configured to estimate instants of ECG occurrence from the EMG signal from an EMG sensor. The onset detection module 72 combines (e.g., fuses information) the multiple breathing signatures extracted from the signals of the sensors and the ECG occurrence information in detecting the onset of a spontaneous breath. An example of fusion logic that may be utilized may include utilizing thresholding in combination with a majority-based approach (i.e., where a majority of the signals from the sensors detect the onset of a spontaneous breath). Another example of fusion logic that may be utilized includes thresholding and weighted average. A further example of fusion logic that may be utilized includes maximum likelihood or another confidence metric. An even further example of fusion logic includes the multiplication of EMG signals from multiple locations and pressure/flow signals (or rate of change of pressure/flow). The advantages of utilizing information fusion include reducing false triggers and enabling faster detection of spontaneous breath events.

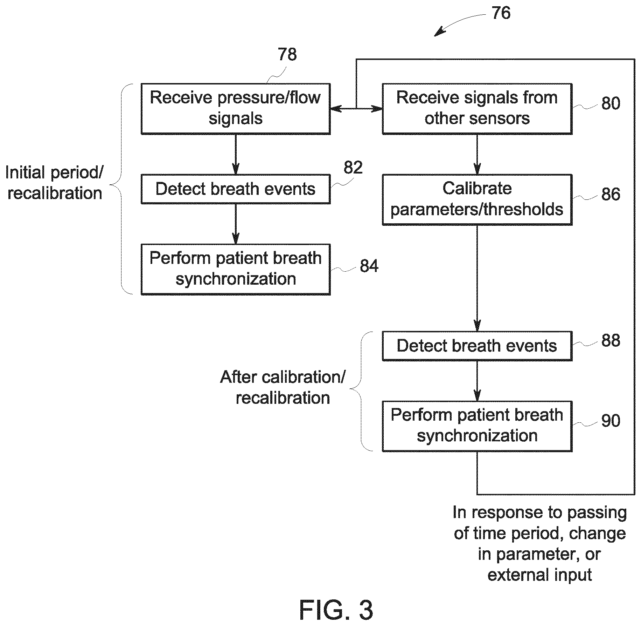

FIG. 3 is a flow chart of a method 76 for patient personalization and real-time adaption for detecting spontaneous breath onset detection (e.g., for a patient coupled to a ventilation system and monitored with noninvasive sensors). The method 76 may be performed by a processing or computing system of a ventilation system (e.g., controller 36 in FIG. 1) or a remote processing or computing system coupled to the controller of ventilation system. Blocks 78-86 of the method 76 may be performed for a set time period (e.g., 20 minutes) initially when a patient is coupled to a ventilation system and the various sensors (e.g., pressure sensor, flow sensor, EMG sensor, etc.) or during recalibration in response to reaching the time for a scheduled recalibration (e.g., 2 hours), a change (e.g., significant change that exceeds a particular threshold) in a physiological parameter, or a received external input. The method 76 includes receiving a pressure signal and a flow signal from a pressure sensor and a flow sensor, respectively, coupled to the patient (block 78). The method 76 also includes receiving signals from one or more sensors (one or more EMG sensors, inductance sensor, piezoelectric sensor, etc.) coupled to the patient that measure different physiological parameters from the pressure sensor and the flow sensor (block 80). The method 76 further includes detecting the onset of the spontaneous breath (e.g., breath event) by the patient based on the pressure signal and the flow signal (block 82). The method 76 still further includes synchronizing providing breathing support to the patient via the ventilation system with the onset of the spontaneous breath detected utilizing the pressure signal and the flow signal (block 84). The method 76 yet further includes while utilizing the pressure signal and the flow signal to synchronize providing breathing support, calibrating or determining parameters and thresholds to be utilized in detecting the onset of the spontaneous breath based on the signals from the one or more sensors (block 86). After calibration or recalibration, the method 76 even further includes after calibration, switching to: detecting the onset of the spontaneous breath (e.g., breath event) by the patient based on the signals from the one or more sensors (block 88) and synchronizing providing breathing support to the patient via the ventilation system with the onset of the spontaneous breath detected utilizing the signals from the one or more sensors (block 90). As noted above, the triggering of providing breath support may be in response to a single signal from a single sensor detecting an onset event or signals from multiple sensors each detecting an onset event.

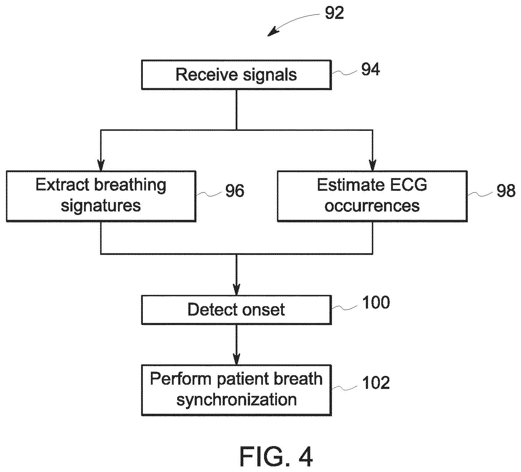

FIG. 4 is a flow chart of a method 92 for utilizing breathing signatures and estimating ECG occurrences in detecting onset of a spontaneous breath by a patient. The method 76 may be performed by a processing or computing system of a ventilation system (e.g., controller 36 in FIG. 1) or a remote processing or computing system coupled to the controller of ventilation system. The method 92 includes receiving respective signals from a flow sensor and/or a pressure sensor, and at least one sensor which provides physiological signals different form the flow and pressure signals (e.g., an EMG sensor) coupled to a patient coupled to a ventilation system (e.g., ventilator) (block 94). The method 92 also includes extracting a breathing signature from a respective signal of each sensor of the plurality of sensors (block 96). For example, a breathing signal may be extracted from each of the pressure/flow signals and the EMG signal. The method 92 further includes estimating ECG occurrences in the EMG signal received from the at least one EMG sensor (block 98). The method 92 still further includes detecting onset of a spontaneous breath of the patient based on the breathing signatures extracted from the respective signals of the plurality of sensors and the estimated ECG occurrences (block 100). The method 92 even further includes patient breath synchronization based on the detected onset of the spontaneous breath to provide breathing support to the patient via the ventilation system (block 102).

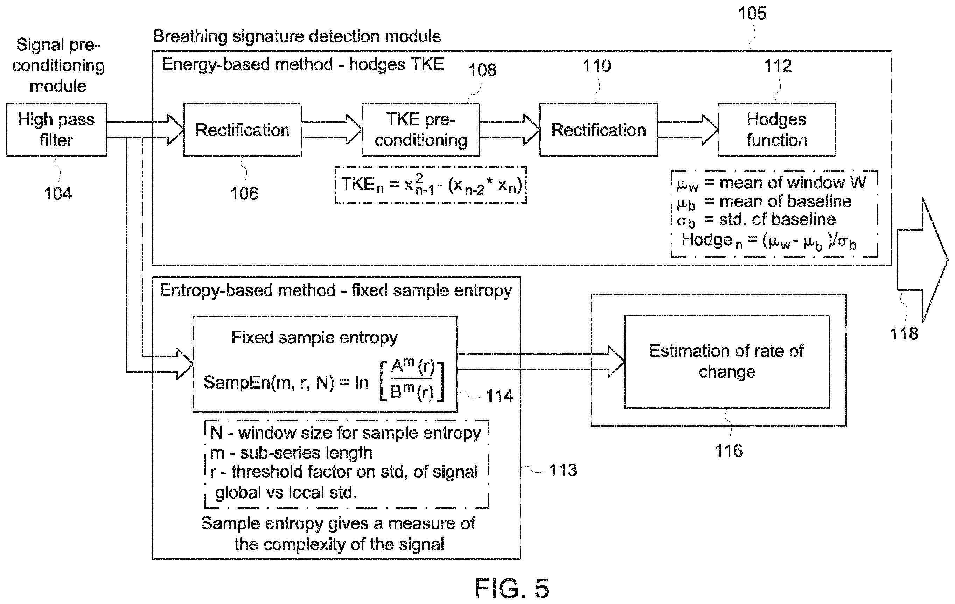

FIG. 5 is a schematic diagram of a different processes for extracting a breathing signature from a signal. The processes may be performed by a processing or computing system of a ventilation system (e.g., controller 36 in FIG. 1) or a remote processing or computing system coupled to the controller of ventilation system. For example, the signal may be an EMG signal and the breathing signature may be an EMG envelope. The signal may be subject to high pass filter 104 by a signal pre-conditioning module (e.g., signal pre-conditioning module 68 in FIG. 2) and the filtered signal passed on to a breathing signature detection module (e.g., breathing signature detection module 74 in FIG. 2) for extracting the breathing signature. In an energy-based method (e.g., Hodges TKE) 105, the filtered signal is sequentially subject to rectification (block 106), TKE pre-conditioning (block 108), rectification (block 110), and Hodges function (block 112). In an entropy-based method (e.g., fixed sample entropy), the filtered signal is subject to a fixed sample entropy algorithm (block 114) from which a rate of change (block 116) may be utilized. The breathing signatures are then passed on to an onset trigger detection module as indicated by arrow 118.

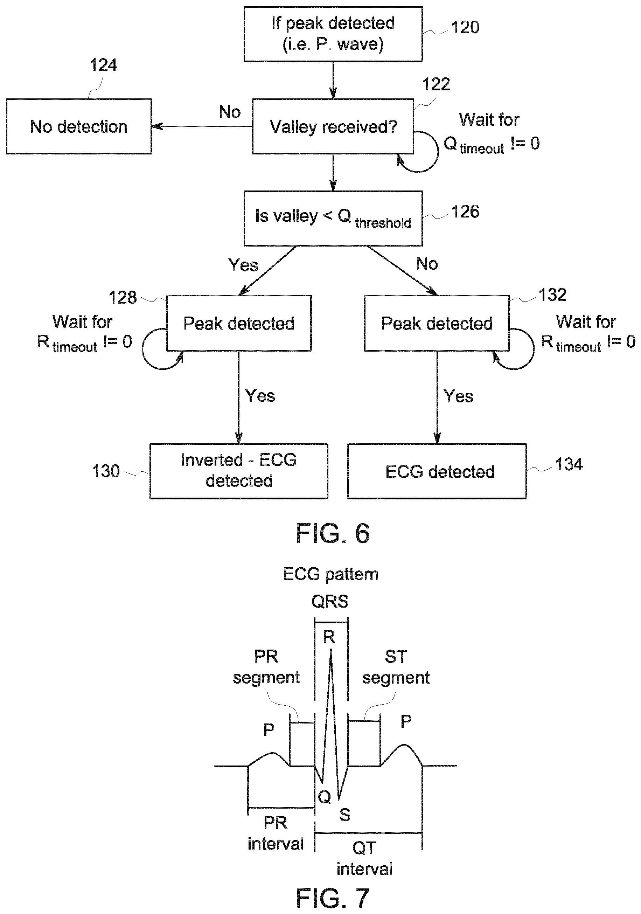

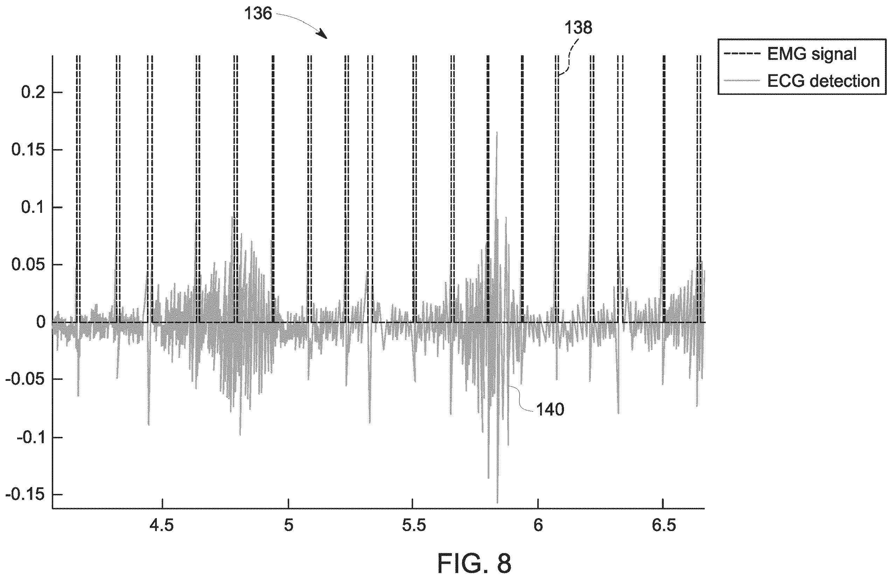

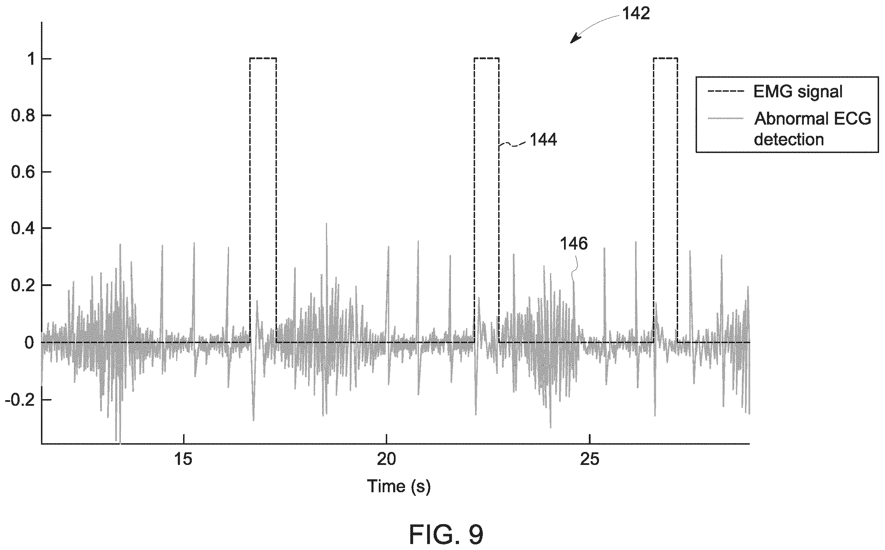

As noted above, in embodiments where the breathing signature is extracted from signals, the onset trigger detection module may determine ECG occurrences. FIG. 6 is a schematic diagram of a process for detecting ECG occurrences and abnormalities (e.g., inverted ECG). The process may be performed by a processing or computing system of a ventilation system (e.g., controller 36 in FIG. 1) or a remote processing or computing system coupled to the controller of ventilation system. If peak (i.e., P complex) (see FIG. 7) is detected in the signal (block 120), the process includes determining if a valley (i.e., Q in QRS complex, see FIG. 7) is received (block 122). If no detection of a valley occur, no ECG is detected (block 124). If a valley is received, the process includes determining if the valley is less than a $Q_{threshold}$ (block 126). If the valley is lower, then the process includes determining if a peak (i.e., R of QRS complex, see FIG. 7) is detected (block 128). If the peak is detected, then an abnormality (inverted ECG) is detected (block 130). If the valley is not less than the $Q_{threshold}$, then the process includes determining if a peak (i.e., R of QRS complex) is detected (block 132). If the peak is detected, then ECG is detected (block 134). In FIG. 8, graph 136 depicts the ECG occurrences 138 detected in an EMG signal 140. In FIG. 9, graph 142 depicts the abnormal ECG occurrences 144 in an EMG signal 146.

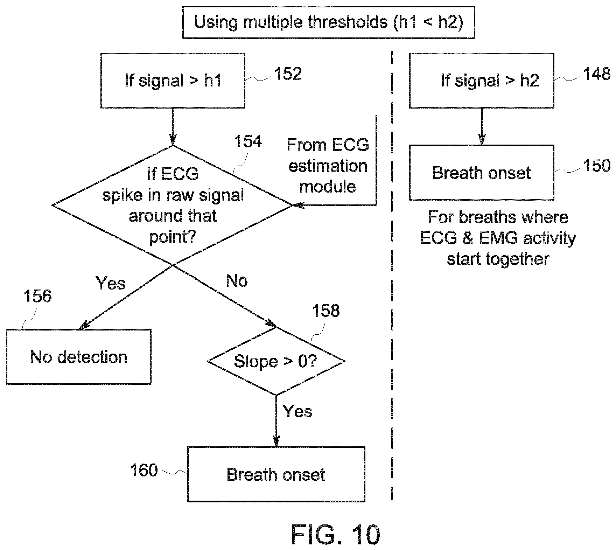

As noted above, the ECG occurrences may be utilized in minimizing false triggers in detecting the onset of spontaneous breaths by the patient. FIG. 10 is a schematic diagram of a process for detecting onset of a spontaneous breath in a signal (e.g., EMG signal) utilizing ECG information. The process may be performed by a processing or computing system of a ventilation system (e.g., controller 36 in FIG. 1) or a remote processing or computing system coupled to the controller of ventilation system. As depicted in FIG. 10, multiple thresholds (h1 and h2) may be utilized in the detecting the onset a spontaneous breath, where threshold h1 is less than threshold h2. The lower threshold h1 improves onset detection time. On the right side of FIG 10, if the signal is greater than h2 (block 148), then onset of a spontaneous breath is definitely detected (block 150). On the left side of FIG. 10, if the signal is greater than threshold h1 but less than h2 (block 152), the ECG information is utilized to determine if an ECG spike in the raw signal is present around that point in the signal (block 154). The ECG occurrences provides the enables a determination to be made if a detected onset is due to breathing or ECG activity. If there is an ECG spike present at that point, then no onset of breath detected (block 156). If there is not an ECG spike present at that point, then it is determined if the slope is greater than zero (block 158). If the slope is greater than zero then onset of a spontaneous breath is detected (block 160).

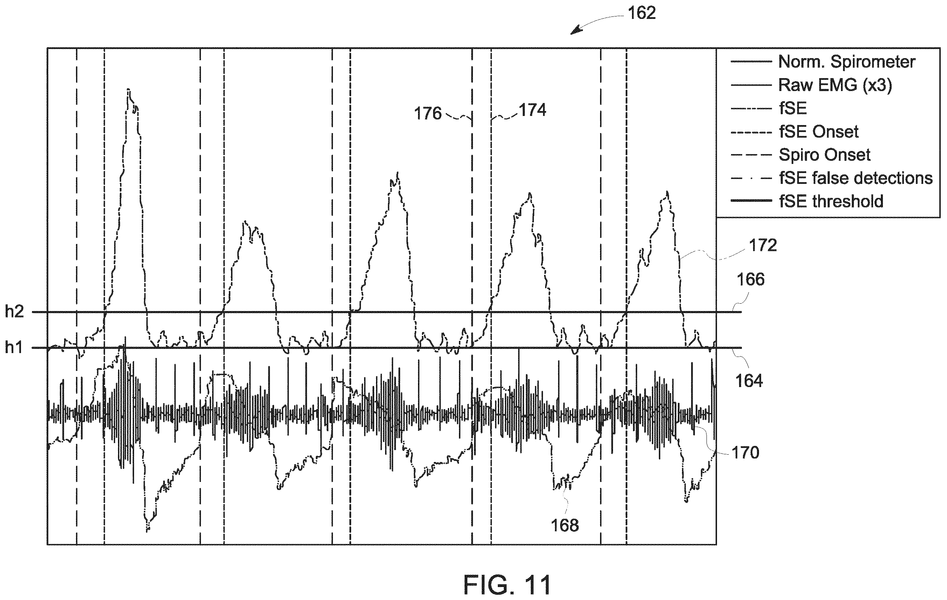

FIG. 11 is a graph 162 illustrating detecting onset of a spontaneous breath utilizing multiple thresholds 164 (h1) and 166 (h2) and ECG information. Plot 168 represents the normalized signal from a spirometer coupled to a patient. Plot 170 represents the raw EMG signal (multiplied by 3) from an EMG sensor coupled to a patient. Plot 172 represents the breathing signature (fixed sample entropy) extracted from the EMG signal. Plots 174 and 176 represents onset of a spontaneous breath detected in the breathing signature (fixed sample entropy) and the signal from the spirometer, respectively, as detected utilizing the thresholds 164, 166. As indicated, the utilization of ECG information as described in FIG. 10, minimized any false triggers as none are present in the graph 162 in FIG 11.

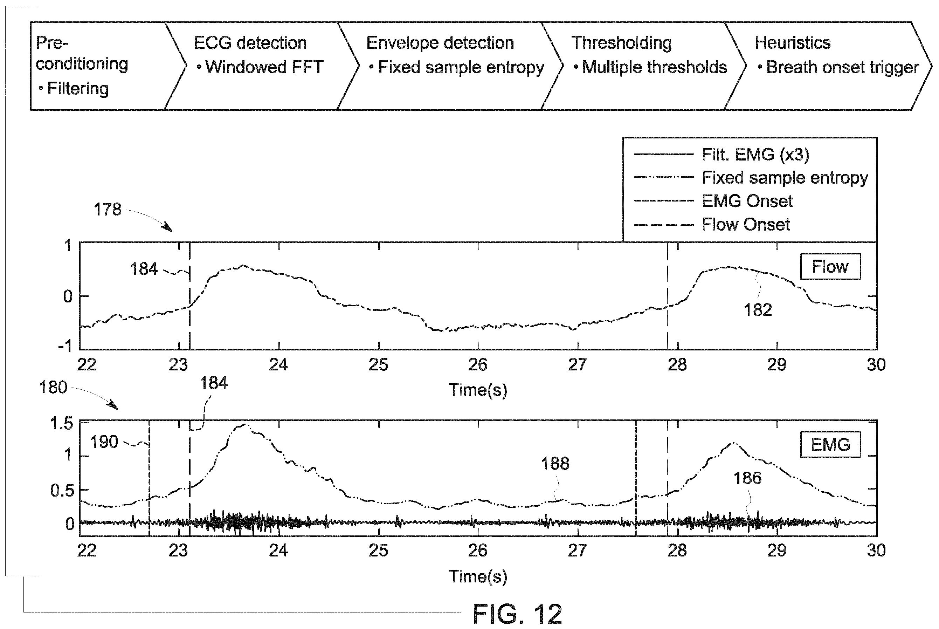

FIG. 12 are graphs 178, 180 illustrating detecting onset of a spontaneous breath in a flow signal compared to an EMG signal. Plot 182 represents the flow signal (e.g., from a spirometer coupled to a patient) in graph 178 over time. Line 184 represents the onset of a spontaneous breath in the flow signal. Plot 186 represents the filtered EMG signal (e.g., obtained from a surface EMG sensor coupled to the patient) in graph 180 over time. Plot 188 represents the fixed sample entropy of the EMG signal in graph 180 over time. Line 190 represents the onset of a spontaneous breath in the EMG signal. The onset of the spontaneous breath was detected earlier in the EMG signal than the flow signal. In FIG. 12, the EMG signal during pre-conditioning was subject to filtering. For ECG detection in the EMG signal, windowed fast Fourier transform (FFT) was utilized. For envelope detection in the EMG signal, fixed sample entropy was utilized. For thresholding of the EMG signal, multiple thresholds were utilized. Heuristics were utilized for the breath onset trigger for the EMG signal.

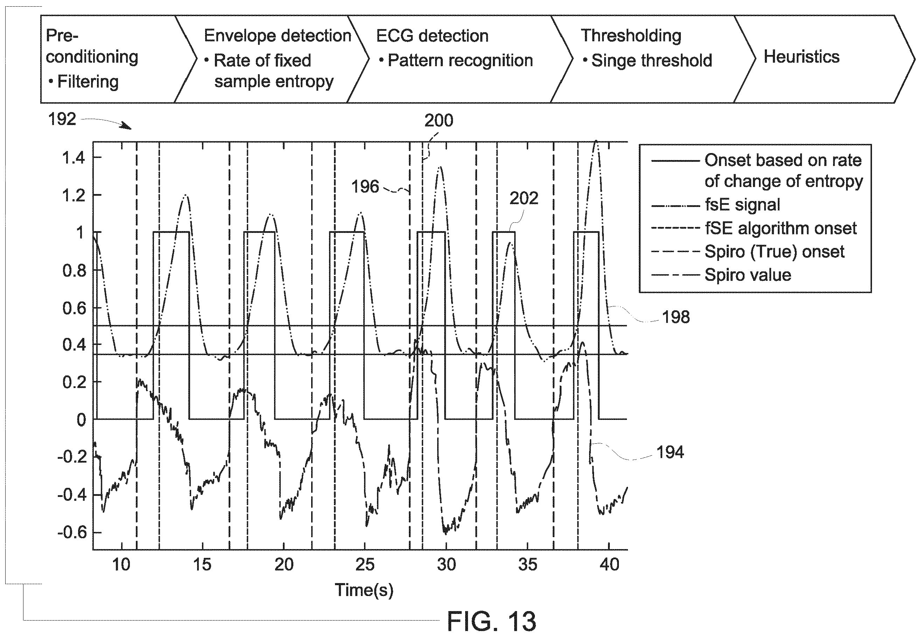

FIG. 13 is a graph 192 illustrating detecting onset of a spontaneous breath in a flow signal compared to an EMG signal. Plot 194 represents the flow signal (e.g., from a spirometer coupled to a patient) over time. Line 196 represents the onset of a spontaneous breath in the flow signal. Plot 198 represents the fixed sample entropy of derived from an EMG signal (e.g., obtained from a surface EMG sensor coupled to the patient) over time. Line 200 represents the onset of a spontaneous breath in the fixed sample entropy. Plot 202 represents the onset of a spontaneous breath based on rate of change (e.g., slope) of entropy in the fixed sample entropy. Since the slope of noise and other disturbances are comparatively low, by setting a threshold on the slope, onset of a spontaneous breath can be detected and the number of false detections reduced further. In FIG. 13, the EMG signal during pre-conditioning was subject to filtering. For envelope detection in the EMG signal, rate of fixed sample entropy was utilized. For ECG detection in the EMG signal, pattern recognition was utilized. For thresholding of the EMG signal, a single threshold was utilized. Heuristics were utilized for the breath onset trigger for the EMG signal.

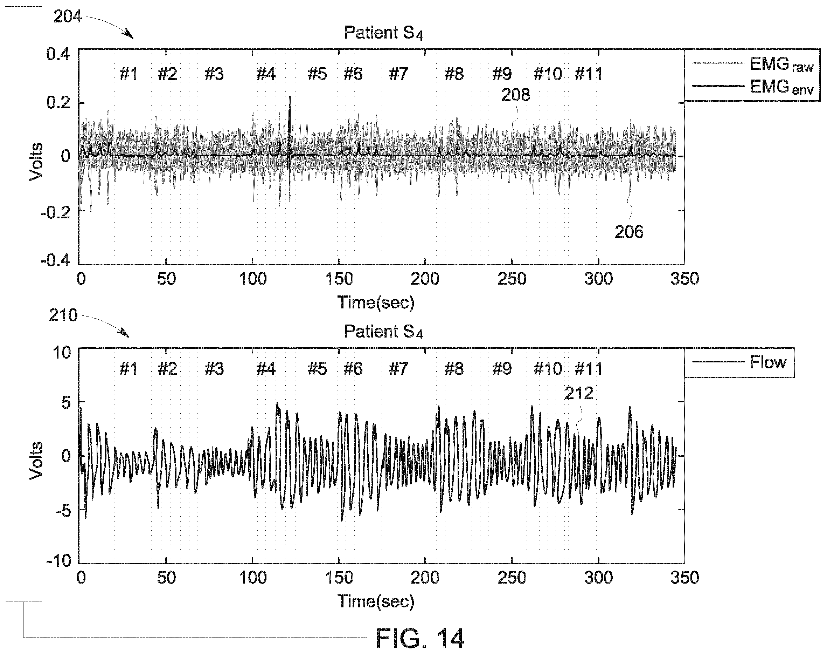

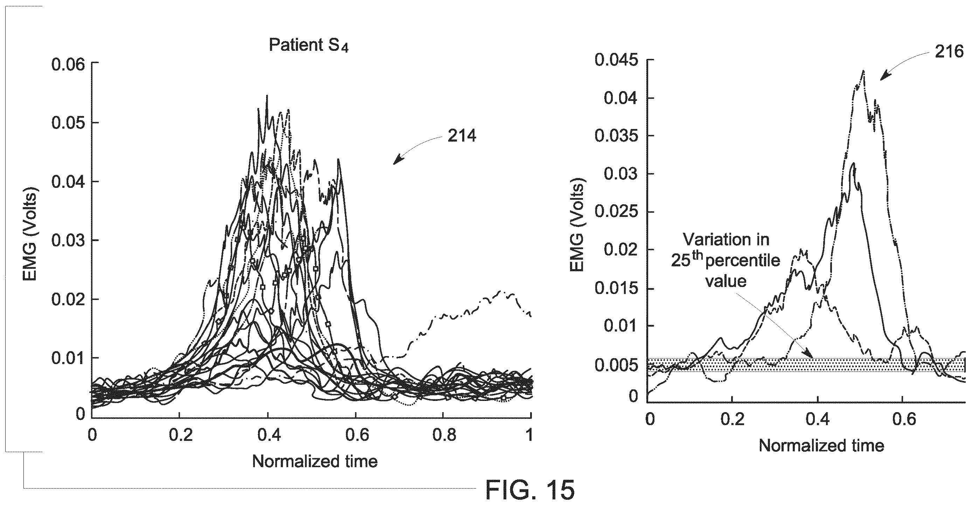

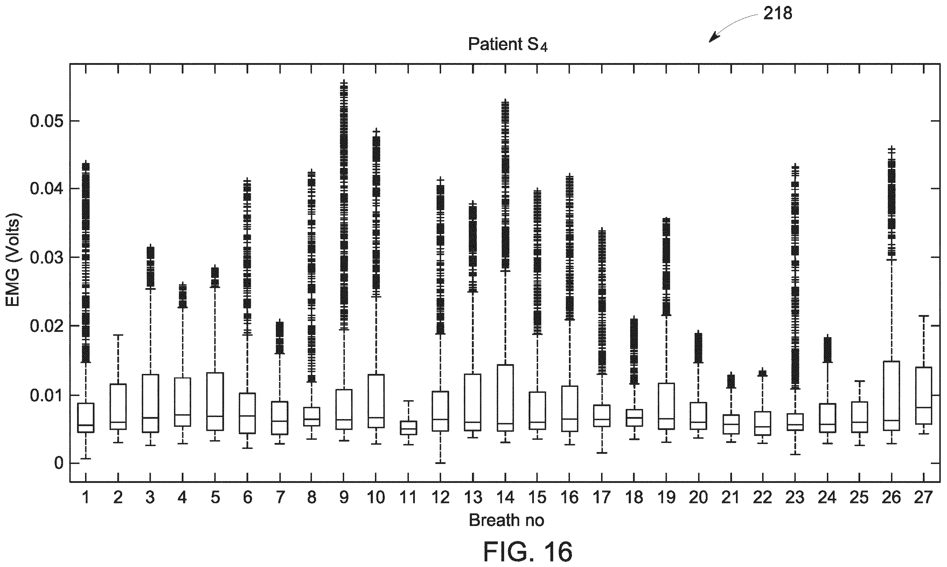

Numerous methods or techniques may be utilized to calibrate thresholds for past breaths to be utilized algorithms for determining onset detection of spontaneous breaths. For example, a statistical method may be utilized. In the statistical method, EMG and pressure or flow data may be collected over a period of time (e.g., 15 minutes) for a calibration dataset. FIG. 14 includes a graph 204 having plots 206 and 208 representing the raw EMG signal and the envelope of the EMG signal collected for calibration data over time. FIG. 14 also includes a graph 210 having a plot 212 representing the flow signal collected for calibration data over time. An EMG processing algorithm (e.g., moving average) with conservative values for the algorithm parameters may be utilized on the EMG data and time normalization of breaths performed. FIG. 15 are examples of graphs 214, 216 for time normalization of the moving average of breaths (e.g., collected for different values for the algorithm parameters). Breath-to-breath variations of a statistics (e.g., $25^{th}$ percentiles of processed EMG in a breath) may be computed as show in the graph 218 of FIG. 16. If the variation in the $25^{th}$ percentile value is within tolerance bounds, the mean of the $25^{th}$ percentile may be utilized as a threshold. If the $25^{th}$ percentile value is not within the tolerance bounds, then breath-to-breath variation may be computed with a lower percentile value.

Besides a statistical method, a modified dynamic threshold method may be utilized for calibrating thresholds from past breaths. Multiple breaths may be utilized in calculating a modified dynamic threshold. In certain embodiments, more weight may be given to more recent breaths in calculating the modified dynamic threshold. In certain embodiments, the modified dynamic threshold may be utilized to calibrate threshold values. As an example using the multiple threshold method along with the dynamic threshold to calibrate the thresholds, the mean of the modified dynamic threshold of past breaths may be utilized to as a lower threshold and the maximum value of the modified dynamic threshold utilized as an upper threshold. In certain embodiments, the dynamic threshold method may be utilized to calibrate a single threshold for a single threshold method. While onset detection of spontaneous breaths is occurring in real time on EMG data (e.g., utilizing a previously calculated modified dynamic threshold), a modified dynamic threshold may be calculated. If a difference in the newly calculated dynamic threshold and previously calculated modified dynamic threshold surpasses a given value the calibration scheduler (e.g., calibration scheduler module 70 in FIG. 2) triggers a calibration event and the calibration module (e.g., calibration module 68 in FIG. 2) may be utilized to update or recalibrate the threshold to be utilized in onset detection.

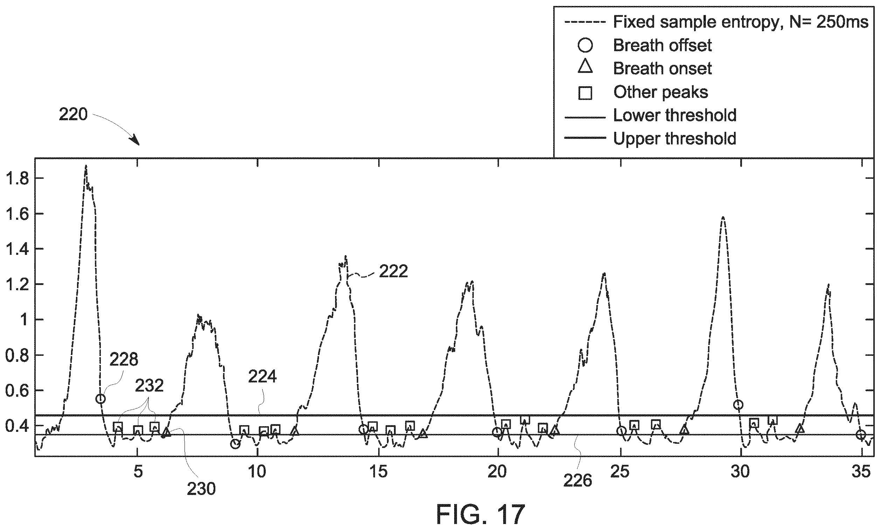

FIG. 17 is a graph 220 illustrating the utilization of modified dynamic thresholds in automated threshold selection on EMG calibration data. Plot 222 represents the fixed sample entropy of an EMG signal. The envelope of the first 30 percent of the breaths are utilized to calibrate the threshold. An upper threshold 224 is set greater than ECG peak amplitudes. A lower threshold 226 is set using the amplitude distribution in the exhalation phase. Points 228 represents breath offset. Point 230 represents breath onset. Points 232 represent other peaks. In certain embodiments, manual threshold selection may be utilized (with similar performance). Thresholds may be automatically set for different subjects and algorithm parameters.

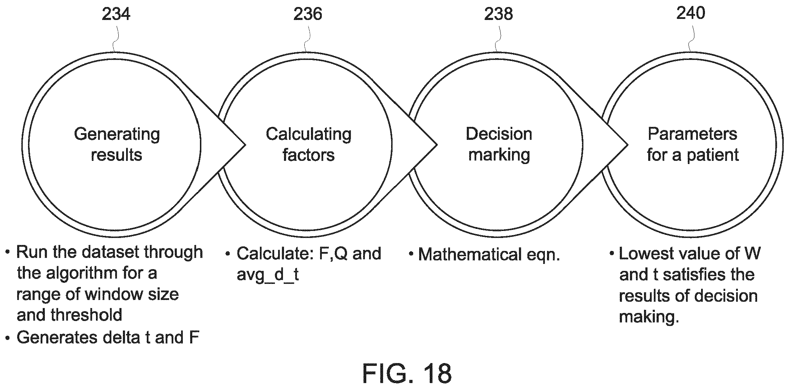

An adaptive approach may be utilized in selecting parameters (which include parameters) for EMG-based algorithms for individual patients in detecting onset of spontaneous breaths. FIG. 18 is a schematic diagram of an adaptive approach for selecting parameters for EMG-based onset detection algorithms for individual patients. The adaptive approach may be performed by a processing or computing system of a ventilation system (e.g., controller 36 in FIG. 1) or a remote processing or computing system coupled to the controller of ventilation system. The first step of the adaptive approach includes generating results 234. For example, initial data (e.g., calibration data) may be gathered from running the EMG-based onset detection algorithm with parameters varied over a range on EMG data gathered from a patient. Examples of important parameters include window size (W) and thresholds (t). At the same time, calibration data may be gathered utilizing a pressure/flow sensor. The adaptive selection of parameters is based on this initial data for the patient. Results may be generated from the result of the algorithm run. For example, a different of time in onset detection ($\Delta t$) between the EMG data utilizing the EMG-based onset detection algorithm and onset detection in the pressure pressure/-flow data.

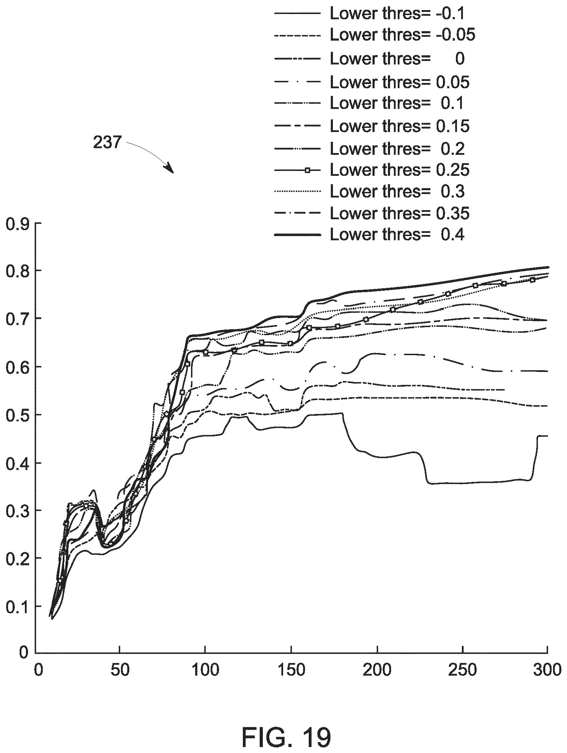

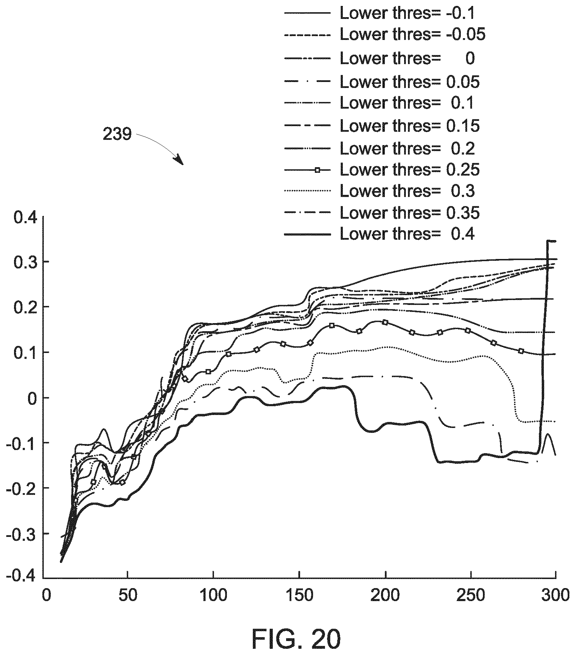

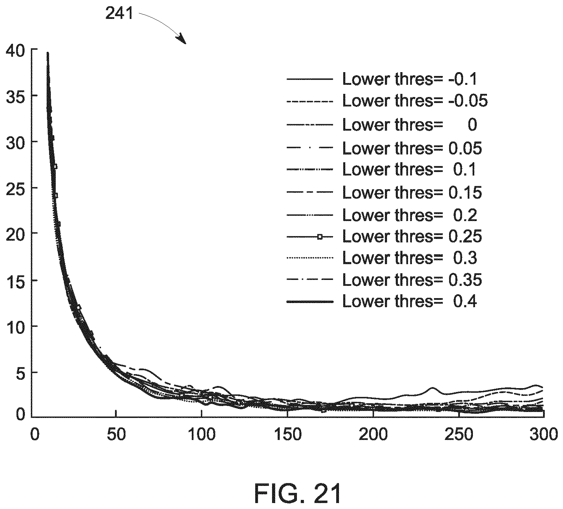

The next step in the adaptive approach includes calculating factors 236 that govern final parameter selection. One factor that is calculated is false breath detection (F). False breath detection is the ratio of false breath detection to true breath detection. Another factor that is calculated average $\Delta t$ (avg. $\Delta t$), which is the average of time difference (between the EMG-based onset detect algorithm and onset detection in the pressure/flow data) in breath onset detection of all the breaths for the patient. A further factor that is calculated in quality factor of a breath (Q). The quality factor of a breath is a quality of a breath with respect to other breaths in the same data set. Normalization of a breath with respect to the other breaths in the same data set also occurs for quality factor of a breath. FIG. 19 is a graph 237 for Q calculated based on initial data collected over a range of values for a parameter (e.g., lower threshold). FIG. 20 is a graph 239 for avg. $\Delta t$ calculated based on initial data collected over a range of values for a parameter (e.g., lower threshold). FIG. 21 is a graph 241 for Q calculated based on initial data collected over a range of values for a parameter (e.g., lower threshold).

After calculating the factors, the adaptive approach includes decision making 238. In decision making, the factors calculated above are plugged into the following decision making equation:

$$\text{Result} = \min(F*(k1*Q + k2*\text{avg. } \Delta t), \qquad\qquad (1)$$

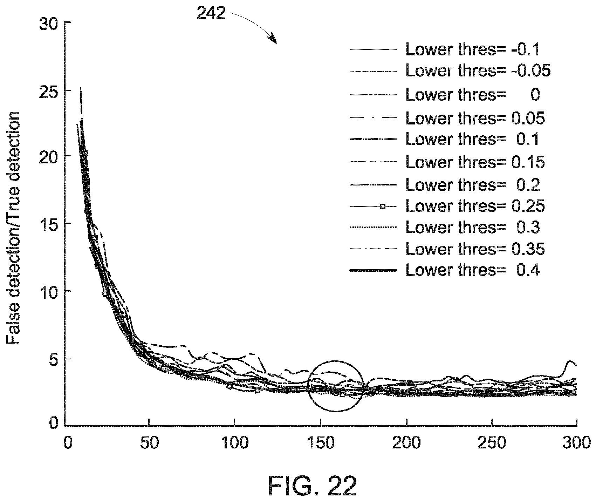

where F equals false detection (W,t)/true detection (W,t), avg. $\Delta t$ equals mean($\Delta t(i)$) for all i (i being a breath), Q equals mean(normalize($\Delta t(i)$)) for all i. Finally, the adaptive approach includes deciding on or selecting parameters for the patient 240 based lowest values of the parameters (e.g., W and t) that satisfies the results of the decisions making (i.e., Equation 1). FIG. 22 is a graph 242 for F versus W over a range of values for a parameter (e.g., lower threshold). $\Delta t$ increases with an increase in W. As depicted in FIG. 22, the number of false detections decreases exponentially with an increase in W. Also, as depicted in FIG. 22, the level of false detections reaches a point 244 where increasing the W does not reduce the false detection ratio.

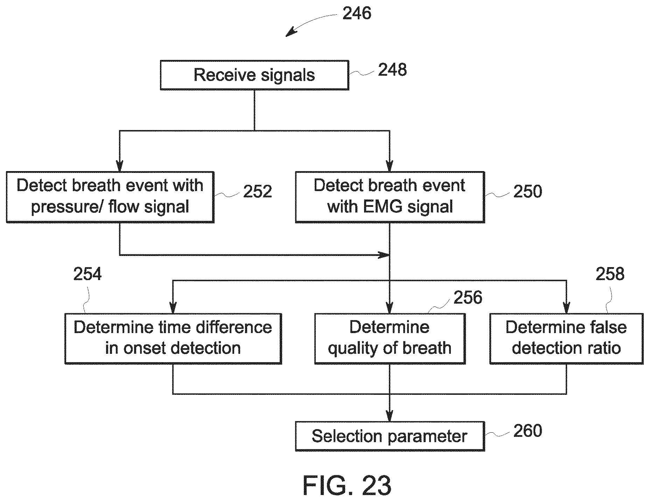

FIG. 23 is flow chart of a method 246 for real-time calibration of parameters utilized in detecting onset of a spontaneous breath of a patient coupled to ventilation system. The method 246 may be performed by a processing or computing system of a ventilation system (e.g., controller 36 in FIG. 1) or a remote processing or computing system coupled to the controller of ventilation system. The method 246 includes receiving respective signals from a flow sensor, a pressure sensor, and at least one EMG sensor coupled to a patient coupled to a ventilation system (e.g., ventilator) (block 248). The method 246 also includes detecting the onset of spontaneous breath utilizing the EMG signal using a range of initial values for at least one parameter (e.g., threshold) for an onset detection algorithm (block 250). The method 246 further includes detecting the onset of spontaneous breath utilizing at least one of the flow signal and the pressure signal (block 252). The method 246 even further includes determining a difference in time ($\Delta t$) in detecting the onset of spontaneous breath in the EMG signal and the at least one of the flow signal and the pressure signal over the initial values for the at least one parameter (block 254). The method 246 still further includes determining a quality of breath (Q) from the EMG signal over the initial values for the at least one parameter (block 256). The method 246 yet further includes determining the false detection ratio (F) in the EMG signal utilizing the EMG data and the pressure/flow data over the initial values for the at least one parameter (block 258). The method 246 even further includes selecting at least one updated parameter (e.g., window size) for the onset detection algorithm to be utilized in detecting the onset of spontaneous breath in the EMG signal based on least the $\Delta t$. The updated parameter may also be based on F and/or Q.

It should be noted that although the various techniques are discussed with regard to signals from EMG signals, the same techniques may be applied to other physiological signals from other sensors (e.g., piezoelectric sensor).

Technical effects of the disclosed embodiments include detecting the onset of spontaneous breath of a patient coupled to a ventilation system (e.g., mechanical ventilator) utilizing onset detection algorithms personalized for the patient that are adapted in real time. The disclosed embodiments may provide for faster and more accurate detection of a patient's effort to breathe. In addition, the disclosed embodiments may improve the synchronization between the ventilator and the patient.

The techniques presented and claimed herein are referenced and applied to material objects and concrete examples of a practical nature that demonstrably improve the present technical field and, as such, are not abstract, intangible or purely theoretical. Further, if any claims appended to the end of this specification contain one or more elements designated as "means for [perform]ing [a function]..." or "step for [perform]ing [a function]...", it is intended that such elements are to be interpreted under 35 U.S.C. 112(f). However, for any claims containing elements designated in any other manner, it is intended that such elements are not to be interpreted under 35 U.S.C. 112(f).

This written description uses examples to disclose the present subject matter, including the best mode, and also to enable any person skilled in the art to practice the invention, including making and using any devices or systems and performing any incorporated methods. The patentable scope of the subject matter is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1.    A ventilation system (10), comprising:

> a plurality of sensors (16, 17, 56, 58, 60, 62) configured to be coupled to a patient and to generate a signal related to a respiratory function of the patient, wherein the plurality of sensors comprises a flow sensor (16), a pressure sensor (17), and at least one electromyography (EMG) sensor (56, 58);
> a memory (46) encoding processor-executable routines;
> a processor (44) configured to access the memory (46) and to execute the processor-executable routines, wherein the routines, when executed by the processor (44), cause the processor (44) to:
>
> > extract a breathing signature from a respective signal of each sensor (16, 17, 56, 58, 60, 62) of the plurality of sensors (16, 17, 56, 58, 60, 62);
> > estimating or measuring electrocardiogram (ECG) occurrences in an EMG signal received from the at least one EMG sensor (56, 58); and
> > detect onset of a spontaneous breath of the patient based on the breathing signatures extracted from the respective signals of the plurality of sensors (16, 17, 56, 58, 60, 62) and the estimated or measured ECG occurrences;
> > **characterized in that**
> > detecting the onset of the spontaneous breath of the patient based on the breathing signatures extracted from the respective signals of the plurality of sensors (16, 17, 56, 58, 60, 62) and the estimated ECG occurrences comprises utilizing the estimated ECG occurrences to determine if a detected onset in one or more of the breathing signatures is due to breathing or an ECG occurrence.

2.    The ventilation system (10) of claim 1, wherein the routines, when executed by the processor, cause the processor to provide breathing support to the patient via the ventilation system (10) upon detecting the onset of the spontaneous breath.

3. The ventilation system (10) of claim 2, wherein providing breathing support to the patient occurs upon detecting the onset of the spontaneous breath in at least two breathing signatures of the respective signals from the plurality of sensors (16, 17, 56, 58, 60, 62).

4. The ventilation system (10) of any of claims 1 to 3, wherein detecting the onset of the spontaneous breath of the patient based on the breathing signatures extracted from the respective signals comprises comparing the breathing signatures to at least one threshold to determine the onset of the spontaneous breath.

5. The ventilation system (10) of claim 4, wherein detecting the onset of the spontaneous breath of the patient based on the breathing signatures extracted from the respective signals comprises determining if the breathing signatures exceed a first threshold, utilizing the estimated ECG occurrences to determine if the detected onsets in the breathing signatures is due to breathing or an ECG occurrence when the breathing signatures exceed the first threshold, and determining the onset of the spontaneous breath is occurring when no ECG occurrences are present where the breathing signatures exceed the first threshold.


**Patentansprüche**

1. Beatmungssystem (10), umfassend:

mehrere Sensoren (16, 17, 56, 58, 60, 62), die dazu ausgelegt sind, an einen Patienten gekoppelt zu werden und ein Signal zu generieren, das sich auf eine Atmungsfunktion des Patienten bezieht, wobei die mehreren Sensoren einen Flusssensor (16), einen Drucksensor (17) und wenigstens einen Elektromyographie (EMG)-Sensor (56, 58) umfasst;
einen Speicher (46), der von einem Prozessor ausführbare Routinen codiert;
einen Prozessor (44), der dazu ausgelegt ist, auf den Speicher (46) zuzugreifen und die von einem Prozessor ausführbaren Routinen auszuführen, wobei die Routinen, wenn sie von dem Prozessor (44) ausgeführt werden, den Prozessor (44) hierzu veranlassen:

Extrahieren einer Atemsignatur aus einem jeweiligen Signal jedes Sensors (16, 17, 56, 58, 60, 62) der mehreren Sensoren (16, 17, 56, 58, 60, 62);
Schätzen oder Messen von Elektrokardiogramm (EKG)-Ereignissen in einem EMG-Signal, das von dem wenigstens einen EMG-Sensor (56, 58) empfangen wird; und
Detektieren des Einsetzens einer Spontanatmung des Patienten basierend auf den Atemsignaturen, die aus den jeweiligen Signalen der mehreren Sensoren (16, 17, 56, 58, 60, 62) extrahiert werden, und den geschätzten oder gemessenen EKG-Ereignissen;
**dadurch gekennzeichnet, dass**
das Detektieren des Einsetzens der Spontanatmung des Patienten, basierend auf den Atemsignaturen, die aus den jeweiligen Signalen der mehreren Sensoren (16, 17, 56, 58, 60, 62) extrahiert werden, und den geschätzten EKG-Ereignissen, das Nutzen der geschätzten EKG-Ereignisse umfasst, um zu bestimmen, ob ein detektiertes Einsetzen in einer oder mehreren der Atemsignaturen auf einen Atemvorgang oder ein EKG-Ereignis zurückzuführen ist.

2. Beatmungssystem (10) nach Anspruch 1, wobei die Routinen, wenn sie von dem Prozessor ausgeführt werden, den Prozessor veranlassen, eine Atemunterstützung für den Patienten über das Beatmungssystem (10) bei Detektieren des Einsetzens der Spontanatmung bereitzustellen.

3. Beatmungssystem (10) nach Anspruch 2, wobei das Bereitstellen einer Atemunterstützung für den Patienten bei Detektieren des Einsetzens der Spontanatmung in wenigstens zwei Atemsignaturen der jeweiligen Signale von den mehreren Sensoren (16, 17, 56, 58, 60, 62) erfolgt.

4. Beatmungssystem (10) nach einem der Ansprüche 1 bis 3, wobei das Detektieren des Einsetzens der Spontanatmung des Patienten basierend auf den Atemsignaturen, die aus den jeweiligen Signalen extrahiert werden, das Vergleichen der Atemsignaturen mit wenigstens einem Schwellwert umfasst, um das Einsetzen der Spontanatmung zu bestimmen.

5. Beatmungssystem (10) nach Anspruch 4, wobei das Detektieren des Einsetzens der Spontanatmung des Patienten basierend auf dem Atemsignaturen, die aus den jeweiligen Signalen extrahiert werden, das Bestimmen umfasst, ob die Atemsignaturen einen ersten Schwellwert überschreiten, das Nutzen der geschätzten EKG-Ereignisse, um zu bestimmen, ob das detektierte Einsetzen in den Atemsignaturen auf einen Atemvorgang oder ein EKG-Ereignis zurückzuführen ist, wenn die Atemsignaturen den ersten Schwellwert überschreiten, und das Bestimmen, dass das Einsetzen der Spontanatmung erfolgt, wenn keine EKG-Ereignisse vorliegen, bei denen die Atemsignaturen den ersten Schwellwert überschreiten.

**Revendications**

1. Système de ventilation (10), comprenant :

   une pluralité de capteurs (16, 17, 56, 58, 60, 62) configurée pour être couplée à un patient et pour générer un signal relatif à une fonction respiratoire du patient, dans lequel la pluralité de capteurs comprend un capteur de débit (16), un capteur de pression (17) et au moins un capteur d'électromyographie (EMG) (56, 58) ;
   une mémoire (46) codant des routines exécutables par processeur ;
   un processeur (44) configuré pour accéder à la mémoire (46) et pour exécuter les routines exécutables par processeur, dans lequel les routines, lorsqu'elles sont exécutées par le processeur (44), amènent le processeur (44) à :

   extraire une signature respiratoire d'un signal respectif de chaque capteur (16, 17, 56, 58, 60, 62) de la pluralité de capteurs (16, 17, 56, 58, 60, 62) ;
   estimer ou mesurer des occurrences d'électrocardiogramme (ECG) dans un signal d'EMG reçu de l'au moins un capteur d'EMG (56, 58) ; et
   détecter le début d'une respiration spontanée du patient sur la base des signatures respiratoires extraites des signaux respectifs de la pluralité de capteurs (16, 17, 56, 58, 60, 62) et des occurrences d'ECG estimées ou mesurées ;
   **caractérisé en ce que**
   la détection du début de la respiration spontanée du patient sur la base des signatures respiratoires extraites des signaux respectifs de la pluralité de capteurs (16, 17, 56, 58, 60, 62) et des occurrences d'ECG estimées comprend l'utilisation des occurrences d'ECG estimées pour déterminer si un début détecté dans une ou plusieurs des signatures respiratoires est dû à la respiration ou à une occurrence d'ECG.

2. Système de ventilation (10) selon la revendication 1, dans lequel les routines, lorsqu'elles sont exécutées par le processeur, amènent le processeur à fournir une assistance respiratoire au patient par le biais du système de ventilation (10) lors de la détection du début de la respiration spontanée.

3. Système de ventilation (10) selon la revendication 2, dans lequel la fourniture d'une assistance respiratoire au patient se produit lors de la détection du début de la respiration spontanée dans au moins deux signatures respiratoires des signaux respectifs provenant de la pluralité de capteurs (16, 17, 56, 58, 60, 62).

4. Système de ventilation (10) selon l'une quelconque des revendications 1 à 3, dans lequel la détection du début de la respiration spontanée du patient sur la base des signatures respiratoires extraites des signaux respectifs comprend la comparaison des signatures respiratoires à au moins un seuil pour déterminer le début de la respiration spontanée.

5. Système de ventilation (10) selon la revendication 4, dans lequel la détection du début de la respiration spontanée du patient sur la base des signatures respiratoires extraites des signaux respectifs comprend la détermination établissant si les signatures respiratoires dépassent un premier seuil, l'utilisation des occurrences d'ECG estimées pour déterminer si les débuts détectés dans les signatures respiratoires sont dûs à la respiration ou à une occurrence d'ECG lorsque les signatures respiratoires dépassent le premier seuil, et la détermination du début de la respiration spontanée se produit lorsqu'aucune occurrence d'ECG n'est présente là où les signatures respiratoires dépassent le premier seuil.

FIG. 1

FIG. 2

EP 4 245 340 B1

76

78

Receive pressure/flow
signals ←——————→ Receive signals from
other sensors          80

Initial period/
recalibration

Detect breath events   82

Calibrate
parameters/thresholds  86

Perform patient breath
synchronization        84

Detect breath events   88

After calibration/
recalibration

Perform patient breath
synchronization        90

In response to passing
of time period, change
in parameter, or
external input

FIG. 3

92

Receive signals — 94

Extract breathing signatures — 96

Estimate ECG occurrences — 98

Detect onset — 100

Perform patient breath synchronization — 102

FIG. 4

FIG. 5

**FIG. 6**

**FIG. 7**

FIG. 8

EMG signal

Abnormal ECG detection

142

144

146

FIG. 9

Time (s)

15   20   25

1   0.8   0.6   0.4   0.2   0   -0.2

Using multiple thresholds (h1 < h2)

If signal > h1 — 152

If signal > h2 — 148

154

From ECG estimation module

If ECG spike in raw signal around that point?

Breath onset — 150

For breaths where ECG & EMG activity start together

Yes

No

156

No detection

158

Slope > 0?

Yes

160

Breath onset

## FIG. 10

FIG. 11

FIG. 12

FIG. 13

FIG. 14

Patient S₄

FIG. 15

Patient S₄

FIG. 16

FIG. 17

234 236 238 240

Generating results

Calculating factors

Decision marking

Parameters for a patient

- Run the dataset through the algorithm for a range of window size and threshold
- Generates delta t and F

- Calculate: F,Q and avg_d_t

- Mathematical eqn.

- Lowest value of W and t satisfies the results of decision making.

FIG. 18

FIG. 19

FIG. 20

FIG. 21

FIG. 22

246

Receive signals ~ 248

Detect breath event with pressure/ flow signal ~ 252

Detect breath event with EMG signal ~ 250

254

Determine time difference in onset detection

256

Determine quality of breath

258

Determine false detection ratio

Selection parameter ~ 260

FIG. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1056499 A
- US 10758693 A
- DE 102015011390
- JP 2019501683 A